Europäisches Patentamt

European Patent Office

Office européen des brevets

(11) Veröffentlichungsnummer: **0 301 280**
**A1**

(12) **EUROPÄISCHE PATENTANMELDUNG**

(21) Anmeldenummer: **88110759.3**

(22) Anmeldetag: **06.07.88**

(51) Int. Cl.⁴: **A61B 5/04**

(30) Priorität: **25.07.87 DE 3724761**

(43) Veröffentlichungstag der Anmeldung:
**01.02.89 Patentblatt 89/05**

(84) Benannte Vertragsstaaten:
**BE DE FR GB NL**

(71) Anmelder: **Richard Wolf GmbH**
**Pforzheimer Strasse 32**
**D-7134 Knittlingen(DE)**

(72) Erfinder: **Ams, Felix, Dipl.-Ing.**
**Gründle Strasse 5**
**D-7539 Kämpfelbach(DE)**
Erfinder: **Vögele, Michael**
**Laierbergstrasse 39**
**D-7539 Kämpfelbach-Ersingen(DE)**

(74) Vertreter: **Wilcken, Thomas, Dipl.-Ing. et al**
**Musterbahn 1**
**D-2400 Lübeck(DE)**

(54) **Video-Endoskop.**

㊼ Video-Endoskop (1) mit einem im Endoskop eingebauten Halbleiter-Bildwandler (3), der das Bild des periodisch mittels einer Beleuchtungseinrichtung (15) mit einer Folge von Teilfarben, beispielsweise durch Verwendung eines Filterrades (18), beleuchteten Objektes aufnimmt und in Teilfarbauszügen des Bildes entsprechende Video-Signal-Komponenten umwandelt, die nacheinander und selektiv nach Teilfarben getrennt in einen Zwischenspeicher eingelesen und anschließend zur Bildung eines Einzelbildes simultan ausgelesen und zur Darstellung des Bildes zu fernsehkompatiblen Video-Signalen aufbereitet werden, bei dem die Lampe (15) der Beleuchtungseinrichtung impulsweise und mit varibablen Impulsamplituden betrieben wird. Der Impulsbetrieb der Lampe (15) erfolgt in Abhängigkeit von der Anzahl der zu erzeugenden Teilfarben mit einem ganzzahligen Vielfachen der Bildwiederholungsfrequenz, wobei der Lichtstrom der von der Lampe (15) abgegebenen Lichtimpulse in Abhängigkeit vom Ist-Wert der jeweiligen Amplituden des Viedeo-Signals veränderbar ist, um in einem vorgegebenen Regelbereich den genannten Ist-Wert auf einen Soll-Wert zu bringen. Im Strahlengang der Lampe (15) ist ein optisches Regelelement (9) angeordnet, das eine weitere Regelung der Lichtintensität übernimmt, sobald der genannte Regelbereich verlassen wird.

FIG. 1

## Video-Endoskop

Die Erfindung betrifft ein Video-Endoskop nach dem Oberbegriff des Anspruches 1.

Moderne Endoskope verwenden zur Generierung von Bildsignalen Halbleiter-Bildwandler. Diese erzeugen aus dem auf ihre Sensor fallenden Licht, das üblicherweise durch einen Lichtleiter zum Endoskop geleitet wird, Signale, die dem Abbild des zu betrachtenden Gegenstandes entsprechen. Diese Signale werden zur Erzeugung von Video-kompatiblen Signalen einer weiteren Verarbeitung unterworfen und schließlich auf einem Fernsehmonitor abgebildet. Zur Erzeugung von Farbbildern mit derartigen Endoskopen ist es beispielsweise aus der DE-PS 34 35 598 bekannt, das Objekt hintereinander mit den drei Primärfarben zu beleuchten, den jeweiligen Farbauszug zwischenzuspeichern und anschließend in der nächsten Halbbild-oder Bildperiode die drei Farbauszüge simultan auszulesen und zu einem fernsehkompatiblen Signal zusammenzusetzen.

Ein Problem bei der Anwendung eines derartigen Endoskopes besteht darin, daß bei der Betrachtung von Objekten mit unterschiedlichem Reflexionsgrad und/oder in unterschiedlicher Entfernung die auf den Bildwandler fallende Lichtmenge zu einer Überstrahlung des Bildwandlers führen kann oder zur Erzeugung eines guten Fernsehbildes nicht mehr ausreicht, wenn sie zu gering ist.

Die aus der DE-PS 34 32 018 bekannte Endoskopanordnung schlägt zur Lösung dieses Problems eine automatische, rein elektronisch arbeitende Helligkeitsregelung durch die Regelung der an einer das Licht aussendenden Lichtquelle liegenden spannung in Abhängigkeit von dem Ergebnis der Integration der aufbereiteten RGB-Ausgangssignale vom Halbleiter-Bildwandler vor. Es hat sich aber gezeigt, daß eine elektronische Regelung allein nicht die für eine gute Ausleuchtung des zu betrachtenden Objektes bei verschiedenen Objektabständen erforderliche hohe Dynamik der Lichtstärke erreicht.

Es ist daher die Aufgabe der vorliegenden Erfindung, ein Video-Endoskop der angegebenen Art so weiterzubilden, daß die Lichtintensität in Abhängigkeit vom betrachteten Objekt mit hinreichender Dynamik so geregelt wird, daß der Halbleiter-Bildwandler einerseits nicht in die Sättigung gerät und andererseits stets ein optimales Videobild erzeugt wird.

Gelöst wird die Aufgabe durch die kennzeichnenden Merkmale des Anspruches 1. Vorteilhafte Weiterbildungen ergeben sich aus den Unteransprüchen.

Neben der Lösung der der Erfindung zugrundeliegenden Aufgabe durch die kombinierte Regelung des Lichtstromes durch eine elektronische Steuerung der Spannung an der Lampe und durch eine mechanische Steuerung eines optischen Regelelementes im Strahlengang des Lichtes hat das Video-Endoskop den Vorteil, daß durch den impulsweisen Betrieb der Lampe deren Lichtausbeute wesentlich höher liegt als im kontinuierlichen Betrieb, wodurch in Kombination mit der zweistufigen Regelung die Dynamik des Lichtstromes erhöht wird.

Die Weiterbildung des Endoskopes gemäß den Ansprüchen 5 und 6 hat darüberhinaus den Vorteil, daß der Motorantrieb des für die sequenzielle Belichtung des Objektes mit den drei Primärfarben vorgesehenen Fiterrades vom Video-Prozessor synchronisiert wird. Dadurch wird die quarzgenaue Synchronisation mehrerer Bildaufnahmegeräte ermöglicht.

Die Erfindung wird anhand der Zeichnungen näher erläutert. Hierbei zeigt:

Figur 1 ein Blockschaltbild für ein Ausführungsbeispiel des erfindungsgemäßen Video-Endoskopes,

Figur 2 ein Ablaufdiagramm, welches die zeitliche Beziehung zwischen dem Belichten des Objektes und dem Abspeichern der vom Bildwandler erzeugten Signale einerseits und der Ansteuerung der Lampe andererseits darstellt,

Figur 3 eine Aufsicht auf ein Filterrad zur Erzeugung der verschiedenfarbigen Farbauszüge des zu betrachtenden Objektes.

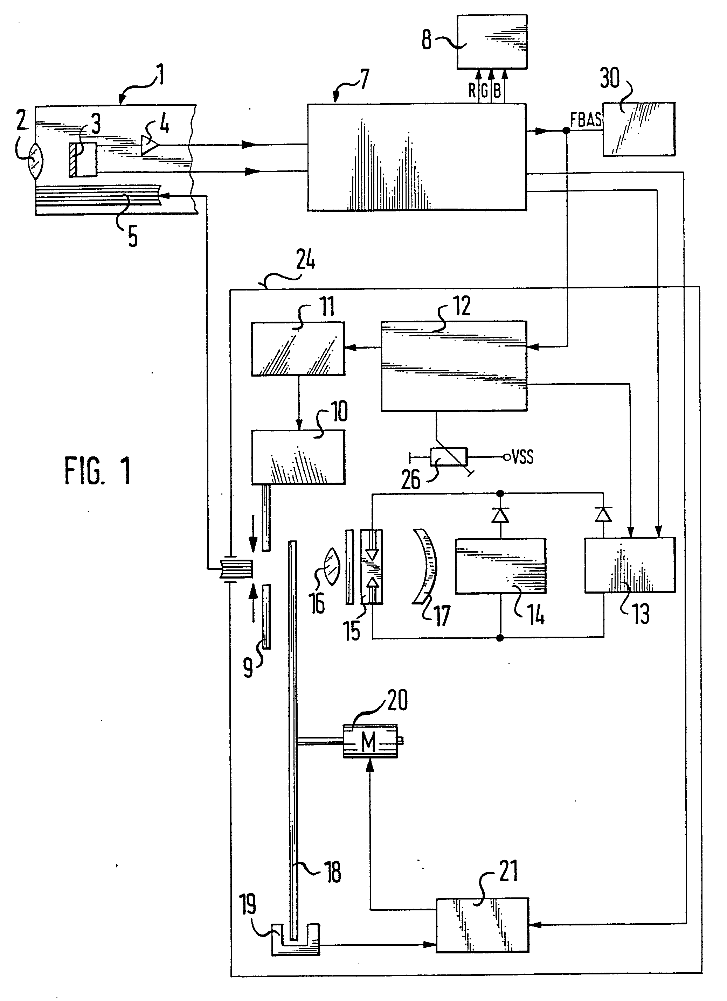

In dem in Figur 1 dargestellten Ausführungsbeispiel des Endoskopes wird ein Abbild eines durch eine Lichtquelle 24 beleuchteten Gegenstandes auf die Oberfläche eines miniarturisierten Halbleiter-Bildwandlers 3 abgebildet. Der Bildwandler 3 ist im Endbereich des Endoskopes 1 mit einer davorliegenden Objektivlinse 2 zum Fokussieren des Bildes positioniert. Er dient zur Umwandlung des Gegenstandsabbildes in elektrische Signale, die über einen Verstärker 4 und einen Video-Prozessor 7 zu fernsehkompatiblen Signalen aufbereitet werden. Die se werden als im Video-Prozessor 7 erzeugte RGB-Signale auf einen RGB Monitor 8 oder als gleichfalls erzeugtes FBAS-Signal auf einen Farbfernsehempfänger 30 geführt, wo sie zur Anzeige gelangen.

Bei in der in Figur 1 gezeigten Anordnung dient eine Edelgasbogenlampe 15 ausgeführte Lichtquelle zur Erzeugung eines zur Betrachtung des Gegenstandes erforderlichen Lichtstromes. Ein Parabolspiegel 17 und eine Sammellinse 16 erhöhen die nutzbare Lichtmenge. Die Lichtzuführung zum Endoskop 2 erfolgt über einen Lichtleiter 5. Ein Filterrad 18 mit den Primärfarben rot, grün, blau liegt im Strahlengang zwischen der Lampe 15 und dem Eintritt in den Lichtleiter 5. Das Filterrad 18 wird von einem Motor 20 nach Erzeugung eines jeden Farbauszuges um einen solchen Winkel gedreht, daß das nächste Filter für die Erzeugung des nächsten Farbauszuges im Strahlengang liegt. Für die Synchronisierung des Motors dient in diesem Ausführungsbeispiel eine Gabellichtschranke 19 und die Motorregelung 21.

Im Strahlengang zwischen dem Filterrad 18 und dem Eintritt in den Lichtleiter 5 ist eine elektrisch betätigte Blende 9 angeordnet, mit der die auf den Gegenstand auftreffende Lichtmenge gesteuert wird. Die Blende 9 wird von einer Blendensteuerung 11 über einen Blendenmotor 10 in der Art gesteuert, daß das FBAS-Signal am Fernsehempfänger 30 auf einen durch einen Einsteller 26 einstellbaren Sollwert ausgeregelt wird, wobei die Regelung von einem Regelkreis 12 vorgenommen wird. Der Regelkreis 12 erzeugt bei Regelabweichungen zwischen Ist- und Sollwert zwei Signale: Ein erstes Signal für eine Lampensteuerung 13, welche die Lichtintensität der Edelgasbogenlampe 15 synchron mit der Erzeugung der einzelnen Farbauszüge hochtastet, und zwar derart, daß die Intensität der Lichtimpulse im Sinne einer linearen Verstärkung oder Schwächung unter Beibehaltung der Amplitudenverhältnisse der Lichtimpulse· im Verhältnis zueinander zur Kompensation der Regelabweichung nachgeregelt wird, und ein zweites Signal, daß an die Blendensteuerung 11 geführt wird.

Wesentlich ist nun, daß das zweite Signal für die Blendensteuerung 11 erst entwickelt wird, wenn die Lampensteuerung 13 nicht mehr in der Lage ist, das FBAS-Signal auf den Sollwert zu regeln. Die Kombination der Regelung des Lichtstromes einerseits durch das Verändern der Lichtintensität der Lampe 15 und andererseits durch das Verstellen der Blende 9 bietet zum einen den Vorzug einer sehr genauen elektronischen Regelung in engen Grenzen und zum anderen eine sehr hohe Dynamik der Lichtstärke (typisch 1 bis 100 %).

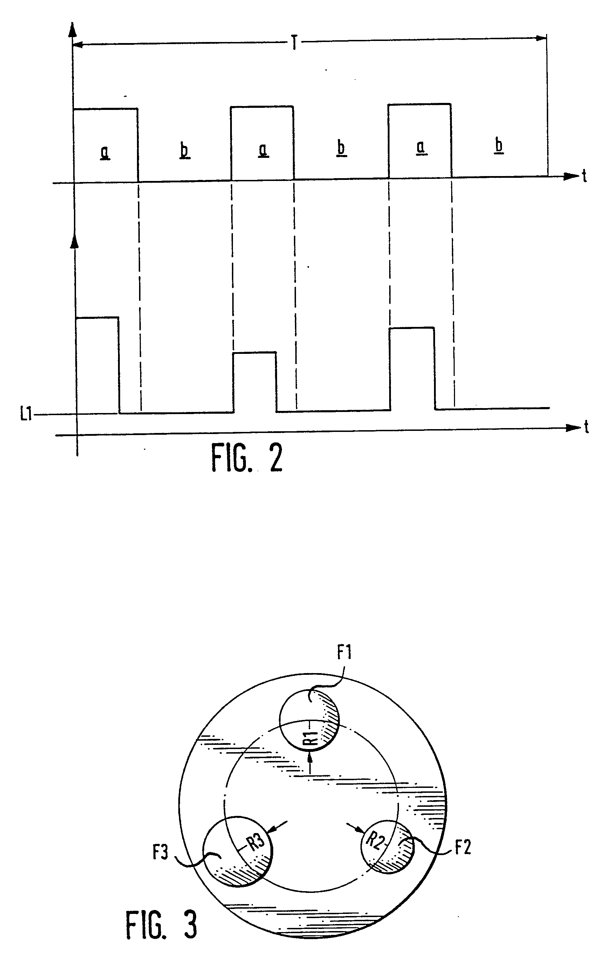

Die zeitliche Relation zwischen den Belichtungen des Gegenstandes mit jeweils einer Farbe, dem Abspeichern der Signale und der Ansteuerung der Lampe 15 ergibt sich aus Figur 2.

Im oberen Teil des Diagramms sind die während einer Bildperiode T auftretenden Belichtungsimpulse dargestellt, die vom Video-Prozessor 7 als Steuersignale dem Halbleiter-Bildwandler 3 zugeführt werden. Ihre Anzahl entspricht der Anzahl der zu erzeugenden Teilfarben, im vorliegenden Ausführungsbeispiel entsprechend den drei Primärfarben. Jeder Belichtungsphase a) folgt eine Abspeicherphase b), während der die vom Bildwandler 3 erzeugten Signale abgespeichert werden. Bei einigen Bildwandlern darf während des Auslesens des Bildinhaltes kein Licht auf dessen Sensorfläche fallen, da dies zu einem Verschmieren des Bildes führen würde. Erreicht wird dies in dem Ausführungsbeispiel während der Auslesephase durch die Verwendung des Filterrades 18, welches während dieser Phase weitergedreht wird, so daß die Filter des Filterrades 18 (Figur 3) dann nicht im Strahlengang liegen. Nach einer Bildperiode werden die abgespeicherten Teilfarbbilder oder Farbauszüge gemeinsam ausgelesen und im Video-Prozessor 7 zu einem Vollfarbbild aufgearbeitet.

Wie im unteren Teil des Diagramms dargestellt, wird die Lampe 15 während jeder Belichtungszeit ausgehend von einer Grundlichtstärke L 1 durch die Lampensteuerung 13 hochgetastet. Die im Ausführungsbeispiel verwendete Edelgasbogenlampe wird beim Einschalten durch die Zündung 14 einmal gezündet und brennt danach dauernd mit einem gewissen Ruhestrom, woraus die im Diagramm bezeichnete Grundlichtstärke resultiert. Während der Integrationszeit des Halbleiter-Bildwandlers 3 wird die Lichtintensität pulsartig in der Amplitude gesteigert, wobei die Amplitude von dem in dem Regelkreis 12 erzeugten ersten Signal abhängt.

Die Lichtmodulation erfolgt demnach mit einem geradzahligen Vielfachen der Bildwiederholungsfrequenz (50 Hertz bei CCIR, 60 Hertz bei EIA) synchron mit dem Belichtungs- und Abspeicherzyklus.

Damit wird der Gesamtleistungsverbrauch gegenüber einer Anwendung mit unmoduliertem Licht bei gleicher Bildhelligkeit gesenkt. Weiterhin resultiert daraus eine höhere Lichtausbeute, da die Lampe im Pulsbetrieb ein mehrfaches an Lichtausbeute liefern kann, als dies im kontinuierlichen Betrieb der Fall wäre, denn im Impulsbetrieb kann sie wesentlich höhere Ströme verkraften. Somit läßt sich der Bildwandler 3 während der Integrationszeit mit einer wesentlich höheren Lichtintensität beaufschlagen als bei einem kontinuierlichen Betrieb der Lampe. Darüberhinaus bietet dieser Betrieb der Lampe die Möglichkeit, die Impulsamplitude während der einzelnen Farbauszüge unterschiedlich hoch zu wählen und so die Transmissionsunterschiede der einzelnen Farbfilter des Filterrades 18 und die spektrale Empfindlichkeit des Bildwandlers 3 auszugleichen. Damit wird erreicht, daß bei einer weißen Bildvorlage der Bildwandler 3 gleichmäßig in allen Farbauszügen belichtet wird, so daß sich für alle derselbe Signal- Rauschabstand ergibt.

Die motorgesteuerte Blende 9 ist vorzugsweise zweistufig ausgeführt in dem Sinne, daß bei einer großen Regelabweichung die Blende mit höherer und bei einer kleinen Regelabweichung mit niedrigerer Geschwindigkeit nachgefahren wird. Dadurch wird zum einen die Trägheit der Lichtregelung reduziert und zum anderen die Schwingneigung des Regelkreises unterdrückt, die sich durch periodisches Auf- und Zufahren der Blende bei gleichem Objektabstand aufgrund eines zu schnellen Motorantriebs bei kleiner Hysterese ausdrücken würde. Anstelle einer mechanischen Blende kann auch ein Graukeilverlaufsfilter verwendet werden, dessen Lage im Strahlengang des Lichtes verstellt wird.

Figur 3 zeigt ein typisches Filterrad mit drei Farbfiltern F, die wie beschrieben beim rotierenden Filterrad nacheinander und periodisch in den Strahlengang der Lampe gebracht werden. Die Radien der Filter R1, R2, R3 sind nicht notwendigerweise gleich. Unterschiedliche Radien können zu einer Anpassung der durchtretenden Lichtmenge an die Sensorcharakteristik des Bildwandlers 3 vorgesehen sein.

Die Filter können sich auch in den Farben von den primären Farben rot, grün, blau unterscheiden. Beispielsweise können die Farben weiß, zyan und gelb verwendet werden. Daraus ergibt sich der Vorteil eines höheren Störsignalabstandes, da die spektralen Durchlaßbereiche dieser Filterkombination größer sind.

Der das Filterrad 18 antreibende Motor ist gemäß einer vorteilhaften Weiterbildung der Erfindung ein Gleichstrommotor. Seine Drehzahl und Phasenlage in bezug auf die ensprechende Teilfarbe wird sychron mit der vom Video-Prozessor 7 bestimmten Wiederholungsfrequenz gesteuert. Der Einsatz von einem Gleichstrommotor vereinfacht somit die Ansteuerung, spart darüberhinaus Platz und senkt die Herstellungskosten.

Die Konzeption des vorliegenden Endoskopes gestattet es zudem daß der Video-Prozessor 7 die gesamte Sychronisation sowohl der Bildaufnahme als auch der Steuerimpulse für den Lampenbetrieb und den Motor des ggf. verwendeten Filterrades übernimmt. Dies ermöglicht die Sychronisation mehrerer Bildaufnahmegeräte. Bei einer Zusammenschaltung mehrerer Bildaufnahmegeräte ist es erforderlich, daß die Geräte in einem Master-Slave-Betrieb sychronisiert sind. Hierbei kann nun das Video-Endoskop sowohl als Master, das heißt als Taktgeber, als auch als Slave betrieben werden, wobei dann ein anderes Bildaufnahmegerät die Sychronisation übernimmt. Durch die genannte Maßnahme ist die Sychronisation darüberhinaus nun nicht mehr von mechanischen Parametern abhängig, da sie nun quarzgenau im Video-Prozessor erzeugt wird. Damit entfallen die bei bekannten Endoskopen eingesetzten Gabellichtschranken, mit Hilfe derer die Sychronisation über das Filterrad vorgenommen wurde.

## Ansprüche

1. Video-Endoskop mit einem im Endoskop eingebauten Halbleiter-Bildwandler, der das Bild des periodisch mittels einer Beleuchtungseinrichtung mit einer Folge von Teilfarben beleuchteten Objektes aufnimmt und in Teilfarbenauszügen des Bildes entsprechende Videosignalkomponenten umwandelt, die nacheinander und selektiv nach Teilfarben getrennt in einem Zwischenspeicher eingelesen und anschließend zur Bildung eines Echtzeit-Einzelbildes simultan ausgelesen und zur Darstellung des Einzelbildes mit einem Videoprozessor zu fernsehkompatiblen Videosignalen verarbeitet werden, dadurch gekennzeichnet, daß die Lampe (15) der Beleuchtungseinrichtung (24) impulsweise und mit variablen Impulsamplituden betrieben wird, und zwar in Abhängigkeit von der Anzahl der zu erzeugenden Teilfarben mit einem ganzzahligen Vielfachen der Bildwiederholungsfrequenz, daß der Lichtstrom der von der Lampe (15) abgegebenen Lichtimpulse in Abhängigkeit vom Ist-Wert der jeweiligen Amplituden des Videosignals veränderbar ist, um in einem vorgegebenen Regelbereich den genannten Ist-Wert auf einen gewünschten Soll-Wert zu bringen, und daß im Strahlengang der Lampe (15) ein optisches Regelelement(9),wie eine Blende oder ein Graukeilfilter, angeordnet ist, das eine weitere Regelung der Lichtintensität übernimmt, sobald der vorerwähnte Regelbereich verlassen wird.

2. Video-Endoskop nach Anspruch1, dadurch gekennzeichnet, daß die Lampe (15) während der Pausen zwischen den Lichtimpulsen mit Ruhestrom betrieben wird.

3. Video-Endoskop nach einem der Ansprüche 1 und 2, dadurch gekennzeichnet, daß der Ist-Wert des Videosignals einem Regelkreis (12) zugeführt wird, in dem dieser Ist-Wert mit einem einstellbaren Soll-Wert des Videosignals verglichen wird, daß bei Regelabweichungen zwischen beiden Werten ein erstes Signal entwickelt und an eine Lampensteuerung (13) gegeben wird, um die Intensität der Lichtimpulse im Sinne einer linearen Verstärkung oder Schwächung, wobei die Amplitudenverhältnisse der Lichtimpulse im Verhältnis zueinander erhalten bleiben, zwecks Kompensation der Regelabweichung nachzuregeln, und daß der Regelkreis (12) ein zweites Signal zur Verstellung des optischen Regelelementes (9) entwickelt, sobald die genannte Kompensation über die Lampensteuerung (13) nicht erreichbar ist.

4. Video-Endoskop nach einem der Ansprüche 1 bis 3, dadurch gekennzeichnet, daß eine als optisches Regelelement (9) verwendete Blende von einem Blendenmotor (10) gesteuert wird, der über eine elektrische Blendensteuerung (11) vom vorerwähnten zweiten Signal des Regelkreises (12) zweistufig gesteuert wird, und zwar bei einer großen Regelabweichung mit höherer und bei einer kleinen Regelabweichung mit niedrigerer Geschwindigkeit.

5. Video-Endoskop nach einem der Ansprüche 1 bis 4, bei dem die Teilfarben zur Beleuchtung des Objektes mit einem Filterrad erzeugt werden, das elektrisch von einem Motor angetrieben wird und mit Filtern ausgestattet ist, die bei rotierendem Filterrad nacheinander und periodisch in den Strahlengang der Lampe bringbar sind, dadurch gekennzeichnet, daß der Motor (20) ein Gleichstrommotor ist, dessen Drehzahl und Phasenlage im bezug auf die entsprechende Teilfarbe sychron mit der vom Video-Prozessor (7) bestimmten Wiederholungsfrequenz gesteuert wird.

6. Video-Endoskop nach einem der Ansprüche 1 bis 5, bei dem der Video-Prozessor den Betrieb des Bildwandlers mit Synchronsignalen steuert, dadurch gekennzeichnet, daß der Video-Prozessor (7) in Abhängigkeit von und sychron mit diesen Synchronsignalen Steuerimpulse für den Lampenbetrieb und den Motor (20) des gegebenenfalls verwendeten Filterrades entwickelt.

FIG. 1

FIG. 2

FIG. 3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.3) |
|---|---|---|---|
| Y | DE-A-3 436 057 (OLYMPUS OPTICAL CO. LTD.) * Seite 9, Zeile 18 - Seite 21, Zeile 18; Figuren 1-7 * | 1,3 | A 61 B 5/04 |
| A | --- | 5,6 | |
| Y | DE-A-3 526 993 (OLYMPUS OPTICAL CO. LTD.) * Seite 11, Zeile 6 - Seite 12, Zeile 28; Figur 3 * | 1,3 | |
| A | --- | 4 | |
| A | EP-A-0 172 680 (OLYMPUS OPTICAL CO. LTD.) * Seite 6, Zeile 6 - Seite 13, Zeile 12; Figuren 3-5 * --- | 1,3,5,6 | |
| A | EP-A-0 078 957 (OLYMPUS OPTICAL CO. LTD.) * Seite 3, Zeile 32 - Seite 5, Zeile 20; Figur 2 * ----- | 1,3,4 | |

| RECHERCHIERTE SACHGEBIETE (Int. Cl.3) |
|---|
| A 61 B 5/00 H 04 N 9/00 H 04 N 3/00 |

Der vorliegende Recherchenbericht wurde für alle Patentansprüche erstellt

| Recherchenort | Abschlußdatum der Recherche | Prüfer |
|---|---|---|
| BERLIN | 14-10-1988 | WEIHS J.A. |